# EUROPEAN PATENT SPECIFICATION

(11) **EP 0 488 195 B1**
(45) Date of publication and mention of the grant of the patent: **31.01.1996**
(21) Application number: 91120243.0
(22) Date of filing: 27.11.1991
(51) Int. Cl.: G01N 33/538, G01N 33/58

(54) **Chemiluminescence immunoassay**
Chemilumineszierender Immunotest
Essai immunologique chemiluminescente

(30) Priority: 29.11.1990 JP 335863/90
(43) Date of publication of application: 03.06.1992
(73) Proprietor: KYOTO DAIICHI KAGAKU CO., LTD., Kyoto-shi Kyoto-fu (JP)
(72) Inventor: Takeuchi, Yoshio, Hikone-shi, Shiga-ken (JP); Katayama, Hisao, Yamatokoriyama-shi, Nara-ken (JP); Machida, Koichi, Ohtsu-shi, Shiga-ken (JP)
(74) Representative: von Kreisler, Alek, Dipl.-Chem.

(56) References cited:
- EP-A- 0 005 271
- EP-A- 0 201 079
- EP-A- 0 303 406
- EP-A- 0 405 578
- WO-A-90/04786
- GB-A- 2 014 727

## Description

### BACKGROUND OF THE INVENTION

### Field of the Invention

The present invention relates to a chemiluminescence immunoassay. More particularly, the present invention relates to an immunoassay which can quickly determine a specific-component in a body fluid, in particular serum, plasma and urine with a high sensitivity. Examples of the specific component in the body fluid includes a tumor marker, a hormone, an antibody, a medicine and a pathogen-originated antigen.

### Description of the Related Art

The immunoassay is classified in a homogeneous method and a heterogeneous method. To determine a minor component such as the tumor marker and the hormone, the highly sensitive heterogeneous method is generally used. In the heterogeneous method, in general, the antigen or the antibody is immobilized on a solid phase and a so-called B/F separation should be carried out. In this method, since one of the antigen and the antibody is immobilized on the solid phase, a reaction rate is smaller than a reaction in a liquid phase, which is a large problem in shortening the reaction time. In clinical tests, it is very important to obtain a test result in a short time for the purpose of shortening a waiting time of a patient and quick diagnosis and therapy by a medical doctor.

To improve the above drawback in the heterogeneous method, Japanese Patent Kokai Publication No. 30963/1987 discloses an immunoassay comprising reacting a sample with a biotin-labeled antibody and a detectably labeled antibody in a liquid phase, capturing an immune complex on a carrier (e.g. polystyrene beads) on which avidin is insolubilized through an avidin-biotin reaction, subjecting the carrier to the B/F separation and detecting the label. However, since this method utilizes the beads, a test tube or a microplate as the solid phase, a surface area of the solid phase is not sufficiently large and a diffusion length is long so that a reaction time to capture the immune complex cannot be shortened. Therefore, this method is still unsatisfactory for accelerating the immunoassay.

Japanese Patent Kokai Publication No. 209370/1989 discloses an immunoassay comprising maintaining a receptor to which an immunologically active material and biotin are bonded, a labeled receptor (each receptor specifically bonds with the immunologically active material) and a biotin-bonded solid phase in a liquid phase at a constant temperature, and then adding avidin so as to immobilize a complex of the immunologically active material and two receptors on the solid phase, whereby an immunoreaction time is greatly shortened.

However, since biotin molecules in the plural complex molecules are bonded to avidin in the liquid phase, the complex molecules are not bonded to the solid phase so that the detection sensitivity is deteriorated.

In general, as a labeling substance, a radioisotope or an enzyme is used. Among them, the enzyme which can be safely handled is preferred. But, in case of the enzyme, a certain time period is necessary for a reaction between the enzyme and a substrate even when any of a colorimetic substrate, a fluorescence substrate and a luminescence substrate is used, and therefore the enzyme interferes with the shortening of the reaction time. As a labeling substance for improving this drawback, there are chemiluminescence materials which do not require amplification by an enzyme, such as acridinium esters, luminol and isoluminol. These compounds are known in the art. For example, the use of the acridinium esters is described in Clin. Chem., 29/8, 1474-1479 (1983) "Acridinium Esters as High-Specific-Activity Labels" in Immunoassay, and their sensitivity is the same as or higher than that of I¹⁵. However, the chemiluminescence substances are highly hydrophobic and are more easily non-specifically absorbed on the solid phase surface than the enzyme. Further, in the method in which avidin is bonded to the solid phase, the solid phase itself emit light. When the solid phase having a large surface area, namely the solid phase to which a larger amount of avidin is bonded, is used, such self-luminescence greatly interferes with the assay.

To quickly determine a specific component in a fluid sample, for example, the body fluid with a high sensitivity, at lease two problems should be solved. The first one relates to quick determination. The reaction rate of the antigen-antibody reaction is highest in the liquid phase. Subsequently, the produced immune complex should be captured by the solid phase for the purpose of the B/F separation. In this step, when the bead, the test tube or the microplate is used as the solid phase as in the conventional methods, the surface area of the solid phase is small and a diffusion length becomes long so that the reaction time is prolonged.

The second problem relates to the sensitivity. To increase the sensitivity of the determination system, it is necessary to suppress the nonspecific absorption of free form antibody or antigen to which the labeling material is bonded onto the solid phase as much as possible. But, as already described, the chemiluminescence compound is highly hydrophobic and more non-specifically absorbed than the enzyme. In the method in which avidin is bonded to the solid phase, after the B/F separation, the solid phase itself emits light when the luminescence from the chemiluminescence compound is induced. They are in particular large problems, when the solid phase having a large surface area is used. Degrees of the nonspecific absorption of the free form labeled antigen or antibody and of the self-luminescence of the solid phase vary with bonding manners of the avidin to the solid phase. With may of usual bonding manners, the nonspecific absorption is high or the solid phase itself emits light. Therefore, the intended results are not obtained.

### BRIEF DESCRIPTION OF THE DRAWINGS

Fig. 1 is the calibration curve for CEA prepared in Example 2,
Figs. 2 and 3 are the time courses for the antigen-antibody reaction and the avidin-biotin reaction, respectively obtained in Example 3,
Figs. 4, 5, 6 and 7 are the calibration curves for AFP (Example 4), insulin (Example 5), T₄ (Example 6) and HBS antibody (Example 7), respectively.

### DETAILED DESCRIPTION OF THE INVENTION

According to the present invention, an aggregate of glass fibers is used as the solid phase for the acceleration of measurement. Thereby, the surface area of the solid phase is greatly increased, the diffusion length is decreased, and the immune complex can be captured by the solid phase through the strong reaction between avidin and biotin. For example, when the immune complex is captured on the solid phase by labeling the antigen or the antibody with biotin and immobilizing avidin on the solid phase, the reaction rate can be the same as that in the liquid phase. As the labeling substance for detection, is used a chemiluminescence compound which emits light immediately after the addition of the luminescence-inducing reagent and does not require the amplification with the enzyme (e.g. acridinium esters, luminol and isoluminol), whereby, the quick determination is achieved.

Herein, the aggregate of glass fiber intends to mean a mass of plural glass fibers, and its shape is not critical. For example, a sheet form glass fiber aggregate such as a glass fiber filter or a ball of the glass fibers may be used. Further, glass fibers contained in a test tube or other container may be used.

According to the present invention, to accelerate the quick antigen-antibody reaction, this reaction is carried out in the liquid phase. That is, in the first embodiment of the present invention, a fluid sample containing an antigen is incubated together with a first antibody which specifically reacts with said antigen and is labeled by biotin and a second antibody.which specifically reacts with said antigen and is labeled by a chemiluminescence compound to form an immune complex comprising said first antibody, said antigen and said second antibody.

In the second embodiment, a fluid sample containing an antigen is incubated together with an antibody which specifically reacts with said antigen and is labeled by one of biotin and a chemiluminescence compound and an antigen which is labeled by the other of biotin and the chemiluminescence compound to effect a competitive antigen-antibody reaction to form an immune complex comprising said labeled antibody and said antigen in the fluid sample or said labeled antigen.

In the third embodiment, a fluid sample containing an antibody is incubated together with an antigen which specifically reacts with said antibody and is labeled by one of biotin and a chemiluminescence compound and an antibody which specifically reacts with said antibody and is labeled by the other of biotin and the chemiluminescence compound to form an immune complex comprising said antigen, said antibody in the sample and said labeled antibody.

In the fourth embodiment, a fluid sample containing an antibody is incubated together with an antigen which specifically reacts with said antibody and is labeled by one of biotin and a chemiluminescence compound and an antibody which specifically reacts with said labeled antigen and is labeled by the other of biotin and a chemiluminescence compound to effect a competitive antigen-antibody reaction to form an immune complex comprising said antigen and said antibody in the sample or said labeled antibody.

Thereafter, to carry out the B/F separation, a mixture containing the formed immune complex is contacted with the glass fiber aggregate to which avidin or streptoavidin and which has a large surface area and good light transparency so as to capture the immune complex through the strong bond between avidin and biotin (a bond constant of 10¹⁵ mole/l).

After effecting the B/F separation with a washing liquid, a luminescence-inducing reagent is applied on the glass fiber aggregate to induce luminescence from the luminescence compound and an intensity of the luminescence is measured.

Among the glass fiber aggregates, the glass fiber filter has a minute network structure of the glass fibers and therefore a large surface area. For example, GA-100 of Toyo Filter Paper Co., Ltd. has an about 100 time larger surface area than a smooth solid surface. Thereby, the diffusion length of the immune complex to the solid phase is greatly reduced and the reaction rate is comparable with that in the liquid phase. In addition, the glass fiber filter has good transparency and, when it contains water, its light transmission is almost 100 %, whereby the luminescence is effectively measured.

Examples of the chemiluminescence compound are acridinium esters, luminol and isoluminol.

When avidin or streptoavidin is bonded to the glass fiber aggregate with conventional glutaraldehyde, there arises some problems such that the bonded parts themselves emit light and an amount of the labeled antibody which is non-specifically absorbed onto the solid surface increases. Since the glass fiber aggregate which has a very large surface area is used, the intensity of luminescence from the solid phase itself and the increase of the non-specifically absorbed antibody have greater influence on the background than in the case where the smooth surface solid phase is used. When the background increases, a S/N ratio decreases and the detection sensitivity decreases.

Hitherto, the bonding manner of the protein and the like to the solid phase has not been studied in view of the chemiluminescence. The bonding manner of avidin or streptoavidin to the glass fiber aggregate has been studied by the present inventors by making reference to a cross linking method of a protein or a peptide or an immobilizing method of an enzyme which are commonly used in the biochemistry field. Degrees of the light emission from the solid phase itself and the nonspecific absorption vary with the bonding methods. Among various bonding methods, two bonding methods achieve small light emission from the solid phase and nonspecific absorption. The method of claims 7 and 8 solves the second problem, namely the nonspecific absorption by these two bonding methods.

One of the most suitable bonding methods comprises treating the glass fiber aggregate with a silane coupling agent having an amino group (e.g. 3-aminopropyltriethoxysilane), reacting biotin to which an N-hydroxysuccinimide group is introduced (e.g. NHS-biotin or a NHS-spacer-biotin complex) with the amino group which is introduced on the glass fiber surface and then reacting avidin or streptoavidin with biotin introduced on the glass fiber surface (hereinafter referred to as "NHS-biotin" method).

In addition to the reduction of the luminescence from the solid phase itself and the nonspecific absorption, the NHS-biotin method greatly increases the reactivity of the immobilized avidin and the biotin-labeled antibody or antigen. When a reaction time of the biotin-labeled antibody or antigen with immobilized avidin by the above bonding method is compared with that with avidin which is immobilized by the conventional method, for example, a method using glutaraldehyde, a time in which the avidin-biotin reaction reaches equilibrium in the case of the above bonding method is less than one tenth of that in the case of the glutaraldehyde method.

One of the reasons for the above result may be as follows:

Since the conventional method for bonding avidin to the solid phase uses the amino groups of avidin randomly, biotin-bonding sites of avidin are modified, so that the reactivity of the avidin with biotin decreases. In the NHS-biotin method, one of the four biotin-bonding sites of avidin is used and then the remaining biotin-bonding sites are not modified, whereby the reactivity of the avidin with biotin does not decrease. Accordingly, the NHS-biotin method contributes not only to the increase of sensitivity of the present invention method but also to the quick determination greatly.

The other of the suitable bonding methods comprising treating the glass fiber aggregate with a silane coupling agent having an amino group (e.g. 3-aminopropyltriethoxysilane), reacting succinic anhydride to the introduced amino group to introduce a carboxyl group, and then reacting N,N'-dicyclohexylcarbodiimide and N-hydroxysuccinimide with the carboxyl group to introduce the NHS group and finally bonding avidin or streptoavidin to the introduced NHS group (hereinafter referred to as "NHS-ester" method).

The background is decreased and the sensitivity is increased by the use of the glass fiber aggregate to which avidin or streptoavidin is bonded by one of the above two bonding methods. Thereby, the quick chemiluminescence immunoassay with high sensitivity is achieved.

The luminescence from the glass fiber filter to which avidin is bonded by various methods and the nonspecific absorption on such glass fiber filter will be shown in following Example 1.

According to the present invention, not only the intact antigen or antibody but also their fragments can be analyzed.

### PREFERRED EMBODIMENTS OF THE INVENTION

The present invention will be illustrated by the following Examples.

### Example 1

### Comparison of luminescence from and nonspecific absorption on avidin-bonded glass fiber filter prepared by various methods

1) NHS-biotin method
   A glass fiber filter (GA-100, a diameter of 13 mm and a thickness of 0.45 mm) was dipped in a 2 % solution of 3-aminopropyltriethoxysilane in acetone at room temperature for one hour to react 3-aminopropyltriethoxysilane with the glass fibers to prepare the glass fiber filter having the bonded amino groups. Then, the amino group-bonded glass fiber filter was dipped in a 0.1 M sodium phosphate buffer (hereinafter referred to as "NaPB") containing 0.0125 mg/ml of NHS-biotin (manufactured by PIERCE) (pH 8.0) at room temperature for one hour. After rinsing with distilled water, the biotin-introduced glass fiber filter was dipped in an avidin solution (an avidin concentration of 0.1 mg/ml, pH of 7.0, 0.1 M, NaPB) at 4°C for at least 30 minutes. A bonding form in this case may be as follow: 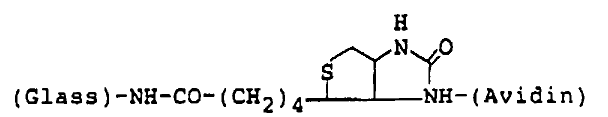
   Using succinimidyl 6-(biotinzamide)hexanate, is formed a complex having a spacer between NHS and biotin having the formula: 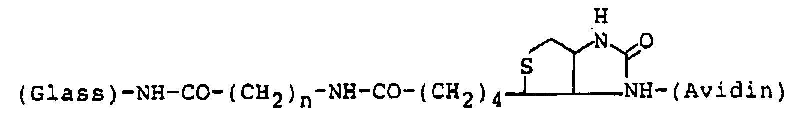 (n = 1 to 5)
2) NHS-ester method
   The amino group-bonded glass fiber filter prepared by the same manner as in the NHS-biotin method was treated with 0.8 M succinic anhydride in saturated sodium tetraborate solution at 4°C for 5 hours. After rinsing with distilled water, the carboxyl group-introduced glass fiber filter was treated in 0.1 M N,N'-dicyclohexylcarbodiimide and 0.1 M N-hydroxysuccinimide in dioxane at room temperature for 2 hours. After rinsing with distilled water, the NHS group-introduced glass fiber filter was dipped in an avidin solution (an avidin concentration of 0.1 mg/ml, pH of 6.5, 0.1M, NaPB) at 4°C overnight. A bonding form in this case may be as follow:

   (Glass)-NH-CO-(CH₂)₂-CO-NH-(Avidin)
3) Glutaraldehyde method (Comparison)
   The amino group-bonded glass fiber filter prepared by the same manner as in the NHS-biotin method was dipped in a 5 % aqueous solution of glutaraldehyde at room temperature for 3 hours. After rinsing with distilled water, the glutaraldehyde-activated glass fiber filter was dipped in an avidin solution (an avidin concentration of 0.1 mg/ml, pH of 10.0, 0.1 M, a carbonate buffer) at 4°C overnight. A bonding form in this case may be as follow:

   (Glass)-N=CH(CH₂)₃CH=N-(Avidin)
4) Tolylene-2,4-diisocyanate (TDIC) method (Comparison)
   The amino group-bonded glass fiber filter prepared by the same manner as in the NHS-biotin method was dipped in a 2 % solution of TDIC (pH 9.5, 0.1 M, a borate buffer) at 0°C for 30 minutes. After rinsing with distilled water, the TDIC-bonded glass fiber filter was dipped in an avidin solution in a borate buffer (an avidin concentration of 0.1 mg/ml) at 4°C overnight. A bonding form in this case may be as follow: 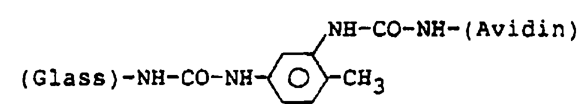
5) Cyanuric chloride method (Comparison)
   The amino group-bonded glass fiber filter prepared by the same manner as in the NHS-biotin method was dipped in a 2 % solution of cyanuric chloride in benzene at room temperature for two hours. After rinsing with distilled water, the cyanuric chloride-bonded glass fiber filter was dipped in an avidin solution (an avidin concentration of 0.1 mg/ml, pH of 8.0, 0.1 M, NaPB) at 4°C overnight. A bonding form in this case may be as follow: 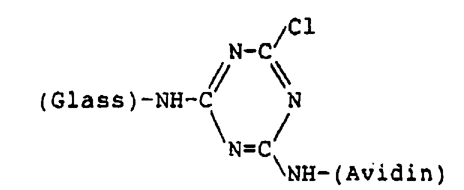
6) Disuccinimidyl sberate (DSS) method (Comparison)
   The amino group-bonded glass fiber filter prepared by the same manner as in the NHS-biotin method was dipped in a 1 mM solution of DSS in DMSO at room temperature for one hour. After rinsing with distilled water, the DSS-bonded glass fiber filter was dipped in an avidin solution (an avidin concentration of 0.1 mg/ml, pH of 7.5, 0.1 M, NaPB) at 4°C overnight. A bonding form in this case may be as follow:

   (Glass)-NH-CO-(CH₂)₆-CO-NH-(Avidin)
7) Hexamethylene diisocyanate method (Comparison)
   The amino group-bonded glass fiber filter prepared by the same manner as in the NHS-biotin method was dipped in a 0.5 % solution of hexamethylene diisocyanate (pH 9.5, 0.1 M, a borate buffer) at room temperature for 2 hours. After rinsing with distilled water, the hexamethylene diisocyanate-bonded glass fiber filter was dipped in an avidin solution (an avidin concentration of 0.1 mg/ml, in a borate buffer) at 4°C for overnight. A bonding form in this case may be as follow:

   (Glass)-NH-CO-NH-(CH₂)₆-NH-CO-NH-(Avidin)

With each of the avidin-bonded glass fiber filters prepared by the methods 1) through 7), an intensity of the luminescence from the glass filter itself and an amount of nonspecific absorption were measured as follows:

An antibody which was labeled with an acridinium ester (4-(2-succinimidiyloxycarbonylethyl)phenyl-10-methylacridinium-9-carboxylate fluorosulfonate: Acridinium I manufactured by Dojin Chemical Co., Ltd.) (1 µg) was added to each avidin-bonded glass fiber filter and reacted for 10 minutes. After rinsing the glass fiber with a phosphate-buffered physiological saline which contained 0.1 % of Tween® 20 (hereinafter referred to as "PBS-Tween"), the residual luminescence from the glass fiber filter was measured. Also, the luminescence from the whole amount of the added labeled antibody (added luminescence) was also measured. The nonspecific absorption was expressed by (residual luminescence/added luminescence) x 100 %.

As a control, with a glass fiber filter having no avidin, the luminescence from the filter itself and the nonspecific absorption were measured. All the glass fiber filters were blocked with a suitable protein.

The luminescence from Acridinium I was measured by adding a 0.3 % solution of hydrogen peroxide in a 1 N aqueous NaOH solution to the glass fiber filter. The intensity of luminescence was measured with Lumiphotometer TD-4000 (manufactured by Laboschience).

The results are shown in the Table.

**Table**

| Method | Luminescence from filter (rlu) | Nonspecific absorption (%) |
|---|---|---|
| Control | 0.05 | 0.0012 |
| 1) | 0.02 | 0.0006 |
| 2) | 0.11 | 0.0015 |
| 3) | 2.14 | 0.031 |
| 4) | 0.84 | 0.057 |
| 5) | 13.3 | 0.10 |
| 6) | 0.83 | 0.019 |
| 7) | 1.3 | 0.018 |

In the cases of the NHS-avidin method and the NHS-ester method according to claims 7 and 8, the luminescence from the filter itself and the nonspecific absorption were in the same levels as in the case of the control filter having no bonded avidin. But, in the comparisons, the luminescence from the filters and the nonspecific absorbance were very high.

In all the cases of the methods 3) through 7), the amount of the nonspecific absorption was much larger than the NHS-biotin method and the NHS-ester method. The reason for this may be the presence of a large amount of hydrophobic groups such as the alkyl chains, the benzene ring or the triazine ring, because the nonspecific absorption occurs mainly through a hydrophobic interaction.

Also, the small amount of a hydrophilic group such as a NH group or a CO group may be a cause for the increase of the nonspecific absorption.

Accordingly, in the NHS-biotin method and the NHS-ester method in which a ratio of the NH group or the CO group to the short alkyl chain is large and the amido bond is formed, luminescence from the filter itself is little and the nonspecific absorption is very small

### Example 2

### Preparation of a calibration curve for CEA (calcinoembryonic antigen) using the avidin-bonded glass fiber filter produced by the NHS-ester method or the Glutaraldehyde method

1) A sample containing CEA in a known concentration (30 µl), 5 µg of a biotin-labeled anti-CEA monoclonal antibody (10 µl) and 0.1 µg of an acridinium ester (Acridinium I)-labeled anti-CEA monoclonal antibody (10 µl) were mixed and incubated at room temperature for five minutes.
2) The above incubated mixture (50 µl) was impregnated in the avidin-bonded glass fiber filter (a diameter of 13 mm and a thickness of 0.45 mm) and incubated at room temperature for ten minutes.
3) After incubation, the glass fiber filter was rinsed with PBS-Tween® for the B/F separation. Then, to the glass filter, a 0.3 % hydrogen peroxide solution (1N aqueous solution of NaOH) as a luminescence-inducing agent to induce luminescence from Acridinium I and the intensity of luminescence was measured with Lumiphotometer TD-4000.

The above measurement was carried out with two kinds of the glass fiber filters produced by the NHS-ester method and the glutaraldehyde method.

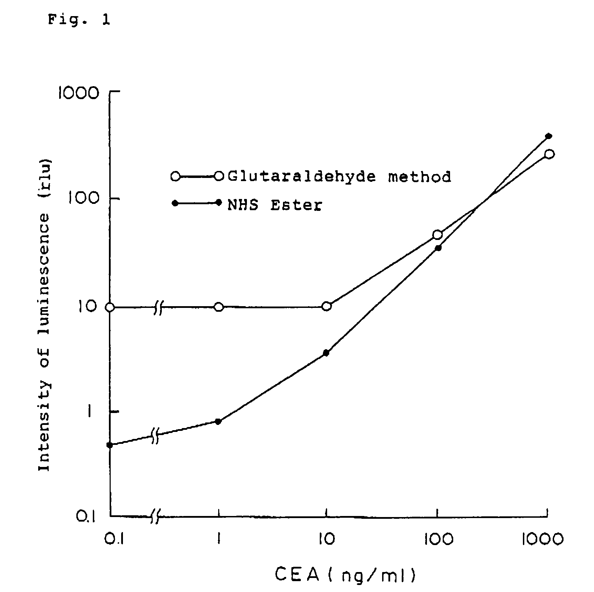

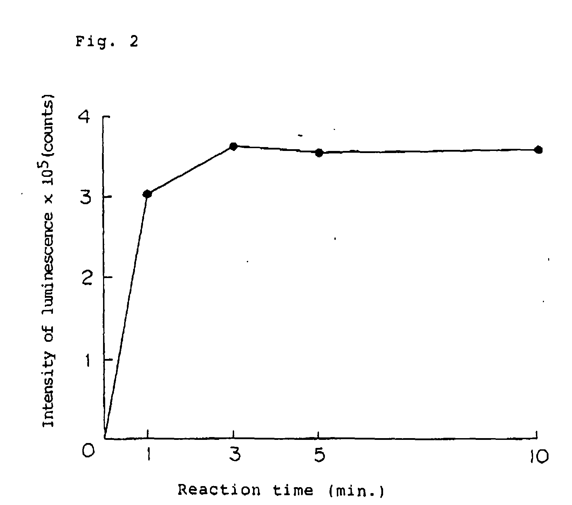

The obtained calibration curves are shown in Fig. 1.

In the case of the glutaraldehyde method, the minimum detection sensitivity was 20 ng/ml, while the NHS-ester method achieved the minimum detection sensitivity of 0.3 ng/ml. These results mean that the NHS-ester method can increase the sensitivity.

### Example 3

### Time courses of the antigen-antibody reaction and the avidin-biotin reaction

A. Time course of the antigen-antibody reaction:
   1) A sample containing AFP (alpha-fetoprotein) in a predetermined concentration (30 µl), 4 µg of a biotin-labeled anti-AFP monoclonal antibody (10 µl) and 0.1 µg of an acridinium ester (Acridinium I)-labeled anti-AFP monoclonal antibody (10 µl) were mixed and reacted at room temperature as time passed.
   2) The reaction liquid (50 µl) was impregnated in the avidin-bonded glass fiber filter (a diameter of 13 mm and a thickness of 0.45 mm) (the NHS-biotin method) and incubated at room temperature for ten minutes.
   3) After incubation, the glass fiber filter was rinsed with FBS-Tween® for the B/F separation. Then, the luminescence from the glass fiber filter was induced in the same manner as in Example 2 and the intensity of luminescence was measured with a photon-counter assembled by Kyoto Daiichikagaku.
B. Time course of the avidin-biotin reaction:
   1) A sample containing AFP in a predetermined concentration (30 µl), 4 µg of a biotin-labeled anti-AFP monoclonal antibody (10 µl) and 0.1 µg of an acridinium ester (Acridinium I)-labeled anti-AFP monoclonal antibody (10 µl) were mixed and incubated at room temperature for ten minutes.
   2) The reaction liquid (50 µl) was impregnated in the avidin-bonded glass fiber filter (the NHS-biotin method) (a diameter of 13 mm and a thickness of 0.45 mm) and incubate at room temperature as time passed.
   3) After incubation, the glass fiber filter was rinsed with PBS-Tween® for the B/F separation. Then, the luminescence was induced and measured in the same manners as above A.

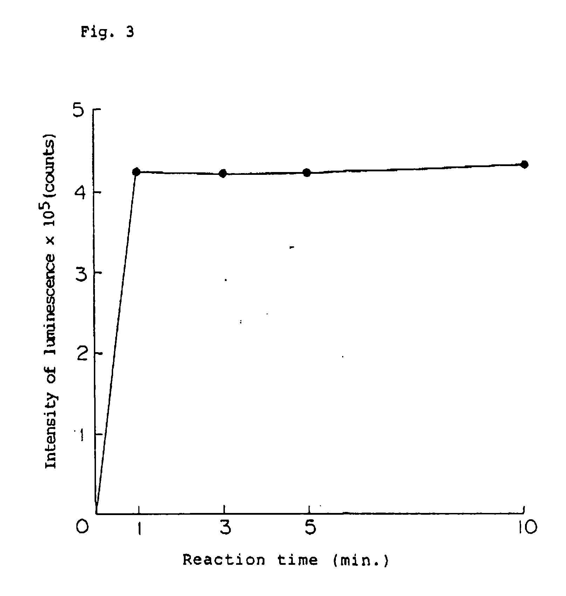

The results are shown in Figs. 2 and 3, respectively.

The antigen-antibody reaction and the avidin-biotin reaction reached equilibrium around 3 minutes and 1 minute, respectively.

In case of the conventional solid phase reactions using the microplate or the beads, it takes several hours till the equilibrium is reached. In contrast, in the present invention, the reaction rate is much increased.

From the above results, it is understood that a very quick immunoassay system can be established, which requires only 3 minutes for the antigen-antibody reaction and only one minute for the avidin-biotin reaction (the measurement of luminescence requiring several seconds).

### Example 4

### Calibration curve for AFP

1) A sample containing AFP in a known concentration (6 µl), PBS (24 µl), 4 µg of a biotin-labeled anti-AFP monoclonal antibody (10 µl) and an acridinium ester (Acridinium I)-labeled anti-AFP monoclonal antibody (10 µl) were mixed and incubated at room temperature for three minutes.
2) The reaction liquid (50 µl) was impregnated in the avidin-bonded glass fiber filter (the NHS-biotin method) (a diameter of 13 mm and a thickness of 0.45 mm) and incubated at room temperature for one minute.
3) After incubation, the glass fiber filter was rinsed with FBS-Tween® for the B/F separation. Then, the luminescence from the glass fiber filter was induced in the same manner as in Example 2 and the intensity of luminescence was measured with a photon-counter assembled by Kyoto Daiichikagaku.

### Example 5

### Calibration curve for insulin

1) A sample containing insulin in a known concentration (10 µl), PBS (20 µl), 0.1 µg of biotin-labeled anti-insulin monoclonal antibody (10 µl) and 0.05 µg of an acridinium ester (Acridinium I)-labeled anti-insulin monoclonal antibody (10 µl) were mixed and incubated at room temperature for three minutes.
2) The reaction liquid (50 µl) was impregnated in the avidin-bonded glass fiber filter (the NHS-biotin method) (a diameter of 13 mm and a thickness of 0.45 mm) and incubated at room temperature for one minute.
3) After incubation, the glass fiber filter was rinsed with PBS-Tween® for the B/F separation. Then, the luminescence from the glass fiber filter was induced in the same manner as in Example 2 and the intensity of luminescence was measured with a photon-counter assembled by Kyoto Daiichikagaku.

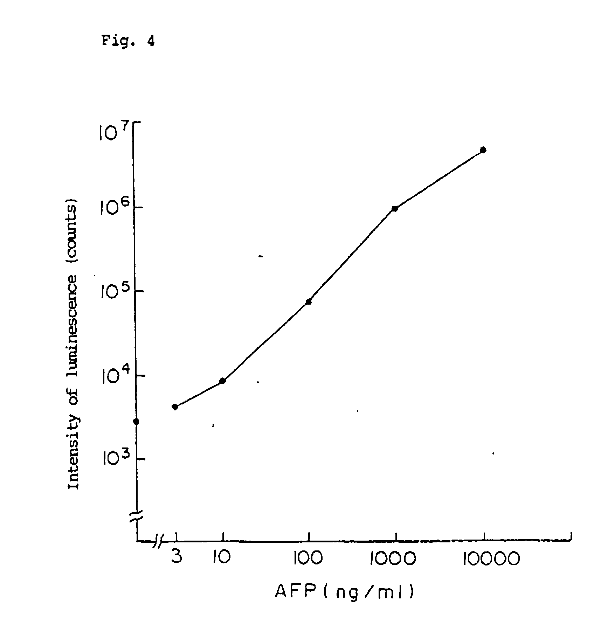

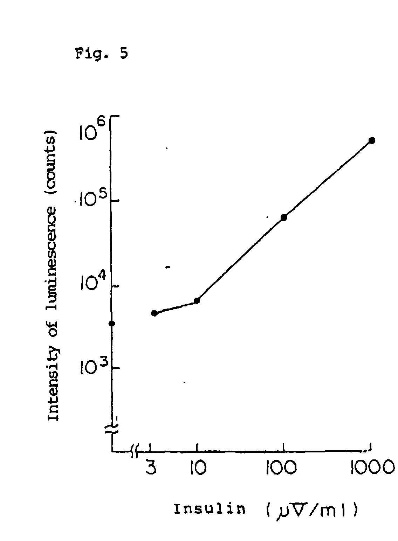

The calibration curves for AFP and insulin are shown in Figs. 4 and 5, respectively.

### Example 6

### Calibration curve for thyroxine

1) A sample containing thyroxine (T₄) in a known concentration (5 µl), PBS containing ANS (8-aniline-1-naphthalenesulfonic acid) (25 µl), 0.1 µg of biotin-labeled anti-T₄ polyclonal antibody (10 µl) and an acridinium ester (Acridinium I)-labeled T₄ (10 µl) were mixed and incubated at room temperature for five minutes.
2) The reaction liquid (50 µl) was impregnated in the avidin-bonded glass fiber filter (the NHS-biotin method) (a diameter of 13 mm and a thickness of 0.45 mm) and incubated at room temperature for one minute.
3) After incubation, the glass fiber filter was rinsed with PBS-Tween for the B/F separation. Then, the luminescence from the glass fiber filter was induced in the same manner as in Example 2 and the intensity of luminescence was measured with a photon-counter assembled by Kyoto Daiichikagaku.

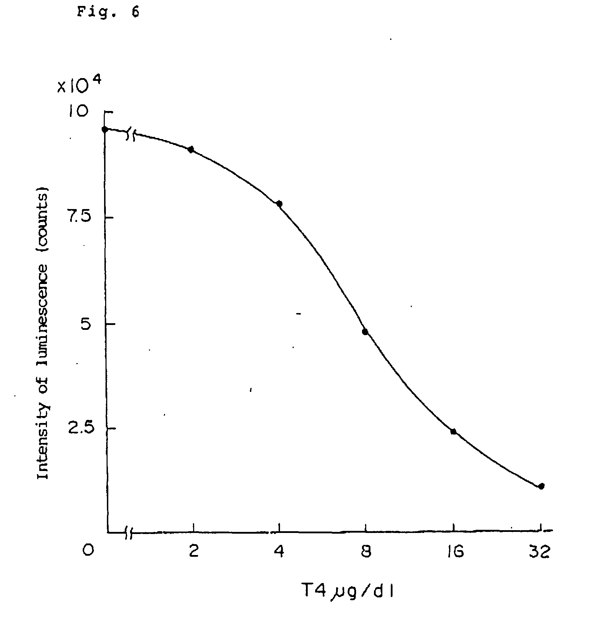

The calibration curve for T₄ is shown in Fig. 6.

### Example 7

### Calibration curve for HBs antibody

1. A sample which was positive against an HBS antibody (diluted with PBS stepwise) (30 µl), a biotin-labeled HBS antibody (10 µl) and an acridinium ester (Acridinium I)-labeled anti-HBS polyclonal antibody (10 µl) were mixed and incubated at room temperature for five minutes.
2) The reaction liquid (50 µl) was impregnated in the avidin-bonded glass fiber filter (a diameter of 13 mm and a thickness of 0.45 mm) and incubated at room temperature for one minute.
3) After incubation, the glass fiber filter was rinsed with PBS-Tween® for the B/F separation. Then, the luminescence from the glass fiber filter was induced in the same manner as in Example 2 and the intensity of luminescence was measured with a photon-counter assembled by Kyoto Daiichikagaku.

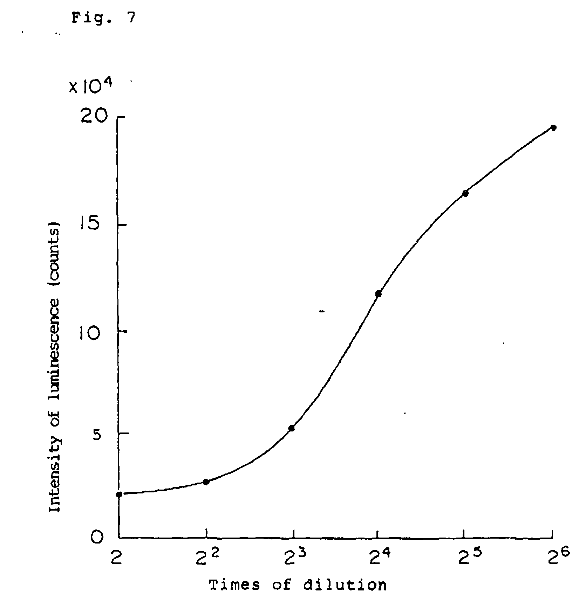

The calibration curve for the HBS antibody is shown in Fig. 7.

## Claims

1. A chemiluminescence immunoassay for determining an antigen in a fluid sample comprising steps of:
(a) incubating said fluid sample together with a first antibody which specifically reacts with said antigen and is labeled by biotin and a second antibody which specifically reacts with said antigen and is labeled by a chemiluminescence compound to form an immune complex comprising said first antibody, said antigen and said second antibody,
(b) impregnating a glass fiber aggregate to which avidin or streptoavidin is bonded with a mixture from the step (a) and incubating it to capture said immune complex on said glass fiber aggregate through an avidin-biotin reaction,
(c) separating said glass fiber aggregate carrying immobilized avidin-immune complex from said mixture,
(d) inducing luminescence from said chemiluminescence compound in the immobilized avidin-immune complex with a luminescence-inducing agent and measuring an intensity of luminescence from said glass fiber aggregate.

2. A chemiluminescence immunoassay for determining an antigen in a fluid sample comprising steps of:
(a) incubating said fluid sample together with an antibody which specifically reacts with said antigen and is labeled by one of biotin and a chemiluminescence compound and an antigen which is labeled by the other of biotin and the chemiluminescence compound to effect a competitive antigen-antibody reaction to form an immune complex comprising said labeled antibody and said antigen in the fluid sample or said labeled antigen,
(b) impregnating a glass fiber aggregate to which avidin or streptoavidin is bonded with a mixture from the step (a) and incubating it to capture said immune complex on said glass fiber aggregate through an avidin-biotin reaction,
(c) separating said glass fiber aggregate carrying immobilized avidin-immune complex from said mixture,
(d) inducing luminescence from said chemiluminescence compound in the immobilized avidin-immune complex with a luminescence-inducing agent and measuring an intensity of luminescence from said glass fiber aggregate.

3. A chemiluminescence immunoassay for determining an antibody in a fluid sample comprising steps of:
(a) incubating said fluid sample together with an antigen which specifically reacts with said antibody and is labeled by one of biotin and a chemiluminescence compound and an antibody which specifically reacts with said antibody and is labeled by the other of biotin and the chemiluminescence compound to form an immune complex comprising said antigen, said antibody in the sample and said labeled antibody,
(b) impregnating a glass fiber aggregate to which avidin or streptoavidin is bonded with a mixture from the step (a) and incubating it to capture said immune complex on said glass fiber aggregate through an avidin-biotin reaction,
(c) separating said glass fiber aggregate carrying immobilized avidin-immune complex from said mixture,
(d) inducing luminescence from said chemiluminescence compound in the immobilized avidin-immune complex with a luminescence-inducing agent and measuring an intensity of luminescence from said glass fiber aggregate.

4. A chemiluminescence immunoassay for determining an antibody in a fluid sample comprising steps of:
(a) incubating said fluid sample together with an antigen which specifically reacts with said antibody and is labeled by one of biotin and a chemiluminescence compound and an antibody which specifically reacts with said labeled antigen and is labeled by the other of biotin and a chemiluminescence compound to effect a competitive antigen-antibody reaction to form an immune complex comprising said antigen and said antibody in the sample or said labeled antibody,
(b) impregnating a glass fiber aggregate to which avidin or streptoavidin is bonded with a mixture from the step (a) and incubating it to capture said immune complex on said glass fiber aggregate through an avidin-biotin reaction,
(c) separating said glass fiber aggregate carrying immobilized avidin-immune complex from said mixture,
(d) inducing luminescence from said chemiluminescence compound in the immobilized avidin-immune complex with a luminescence-inducing agent and measuring an intensity of luminescence from said glass fiber aggregate.

5. A chemiluminescence immunoassay for determining an antibody in a fluid sample comprising steps of:
(a) incubating said fluid sample together with an antibody which specifically reacts with said antibody in said fluid sample and is labeled by biotin and an antigen which specifically reacts with said antibody in said fluid sample and is labeled by a chemiluminescence compound to form an immune complex comprising said labeled antibody, said antibody in the fluid sample and said labeled antigen,
(b) impregnating a glass fiber aggregate to which avidin or streptoavidin is bonded with a mixture from the step (a) and incubating it to capture said immune complex on said glass fiber aggregate through an avidin-biotin reaction,
(c) separating said glass fiber aggregate carrying immobilized avidin-immune complex from said mixture,
(d) inducing luminescence from said chemiluminescence compound in the immobilized avidin-immune complex with a luminescence-inducing agent and measuring an intensity of luminescence from said glass fiber aggregate.

6. A chemiluminescence immunoassay for determining an antibody in a fluid sample comprising steps of:
(a) incubating said fluid sample together with a first antigen which specifically reacts with said antibody in said fluid sample and is labeled by biotin and a second antigen which specifically reacts with said antibody in said fluid sample and is labeled by a chemiluminescence compound to form an immune complex comprising said labeled first antigen, said antibody in the fluid sample and said labeled second antigen,
(b) impregnating a glass fiber aggregate to which avidin or streptoavidin is bonded with a mixture from the step (a) and incubating it to capture said immune complex on said glass fiber aggregate through an avidin-biotin reaction,
(c) separating said glass fiber aggregate carrying immobilized avidin-immune complex from said mixture,
(d) inducing luminescence from said chemiluminescence compound in the immobilized avidin-immune complex with a luminescence-inducing agent and measuring an intensity of luminescence from said glass fiber aggregate.

7. The chemiluminescence immunoassay according to any one of claims 1 to 6, wherein, to said glass fiber aggregate, avidin or streptoavidin is bonded by
(i) providing a glass aggregate having an amino group,
(ii) reacting said amino group with biotin to which an N-hydroxysuccinimide group is bound so as to introduce biotin on said glass fiber, and
(iii) reacting said introduced biotin with avidin or streptoavidin.

8. The chemiluminescence immunoassay according to any one of claims 1 to 6, wherein, to said glass fiber aggregate, avidin or streptoavidin is bonded by
(i) providing a glass aggregate having an amino group,
(ii) reacting said amino group of the glass aggregate with succinic anhydride to introduce a carboxylic group,
(iii) reacting said introduced carboxyl group with dicyclohexylcarbodiimide and N-hydroxysuccinimide so as to introduce an N-hydroxysuccinimide group on said glass fiber, and
(iv) reacting said introduced N-hydroxysuccinimide group with avidin or streptoavidin.

## Patentansprüche

1. Chemilumineszenz-Immunoassay zur Bestimmung eines Antigens in einer flüssigen Probe, umfassend die Schritte:
(a) Inkubieren der flüssigen Probe zusammen mit einem ersten Antikörper, der spezifisch mit dem Antigen reagiert und mit Biotin markiert ist, und einem zweiten Antikörper, der spezifisch mit dem Antigen reagiert und mit einer chemilumineszierenden Verbindung markiert ist, unter Bildung eines Immunkomplexes, der den ersten Antikörper, das Antigen und den zweiten Antikörper umfaßt;
(b) Imprägnieren eines Glasfaseraggregats, an das Avidin oder Streptoavidin gebunden ist, mit einem Gemisch aus Schritt (a) und Inkubieren, um den Immunkomplex durch eine Avidin-Biotin-Reaktion an dem Glasfaseraggregat abzufangen;
(c) Abtrennen des Glasfaseraggregats, das immobilisierten Avidin-Immunkomplex trägt, aus dem Gemisch;
(d) Induzieren von Lumineszenz der chemilumineszierenden Verbindung in dem immobilisierten Avidin-Immunkomplex mit einem lumineszenzinduzierenden Mittel und Messen der Intensität der Lumineszenz des Glasfaseraggregats.

2. Chemilumineszenz-Immunoassay zur Bestimmung eines Antigens in einer flüssigen Probe, umfassend die Schritte:
(a) Inkubieren der flüssigen Probe zusammen mit einem Antikörper, der spezifisch mit dem Antigen reagiert und entweder mit Biotin oder einer chemilumineszierenden Verbindung markiert ist, und einem Antigen, das mit dem anderen, d.h. Biotin oder der chemilumineszierenden Verbindung, markiert ist, so daß eine kompetitive Antigen-Antikörper-Reaktion unter Bildung eines Immunkomplexes, der den markierten Antikörper und das Antigen in der flüssigen Probe oder das markierte Antigen umfaßt, bewirkt wird;
(b) Imprägnieren eines Glasfaseraggregats, an das Avidin oder Streptoavidin gebunden ist, mit einem Gemisch aus Schritt (a) und Inkubieren, um den Immunkomplex durch eine Avidin-Biotin-Reaktion an dem Glasfaseraggregat abzufangen;
(c) Abtrennen des Glasfaseraggregats, das immobilisierten Avidin-Immunkomplex trägt, aus dem Gemisch;
(d) Induzieren von Lumineszenz der chemilumineszierenden Verbindung in dem immobilisierten Avidin-Immunkomplex mit einem lumineszenzinduzierenden Mittel und Messen der Intensität der Lumineszenz des Glasfaseraggregats.

3. Chemilumineszenz-Immunoassay zur Bestimmung eines Antikörpers in einer flüssigen Probe, umfassend die Schritte:
(a) Inkubieren der flüssigen Probe zusammen mit einem Antigen, das spezifisch mit dem Antikörper reagiert und entweder mit Biotin oder einer chemilumineszierenden Verbindung markiert ist, und einem Antikörper, der spezifisch mit dem Antikörper reagiert und mit dem anderen, d.h. Biotin oder der chemilumineszierenden Verbindung, markiert ist, unter Bildung eines Immunkomplexes, der das Antigen, den Antikörper in der Probe und den markierten Antikörper umfaßt;
(b) Imprägnieren eines Glasfaseraggregats, an das Avidin oder Streptoavidin gebunden ist, mit einem Gemisch aus Schritt (a) und Inkubieren, um den Immunkomplex durch eine Avidin-Biotin-Reaktion an dem Glasfaseraggregat abzufangen;
(c) Abtrennen des Glasfaseraggregats, das immobilisierten Avidin-Immunkomplex trägt, aus dem Gemisch;
(d) Induzieren von Lumineszenz der chemilumineszierenden Verbindung in dem immobilisierten Avidin-Immunkomplex mit einem lumineszenzinduzierenden Mittel und Messen der Intensität der Lumineszenz des Glasfaseraggregats.

4. Chemilumineszenz-Immunoassay zur Bestimmung eines Antikörpers in einer flüssigen Probe, umfassend die Schritte:
(a) Inkubieren der flüssigen Probe zusammen mit einem Antigen, das spezifisch mit dem Antikörper reagiert und entweder mit Biotin oder einer chemilumineszierenden Verbindung markiert ist, und einem Antikörper, der spezifisch mit dem markierten Antigen reagiert und mit dem anderen, d.h. Biotin oder der chemilumineszierenden Verbindung, markiert ist, so daß eine kompetitive Antigen-Antikörper-Reaktion unter Bildung eines Immunkomplexes, der das Antigen und den Antikörper in der Probe oder den markierten Antikörper umfaßt, bewirkt wird;
(b) Imprägnieren eines Glasfaseraggregats, an das Avidin oder Streptoavidin gebunden ist, mit einem Gemisch aus Schritt (a) und Inkubieren, um den Immunkomplex durch eine Avidin-Biotin-Reaktion an dem Glasfaseraggregat abzufangen;
(c) Abtrennen des Glasfaseraggregats, das immobilisierten Avidin-Immunkomplex trägt, aus dem Gemisch;
(d) Induzieren von Lumineszenz der chemilumineszierenden Verbindung in dem immobilisierten Avidin-Immunkomplex mit einem lumineszenzinduzierenden Mittel und Messen der Intensität der Lumineszenz des Glasfaseraggregats.

5. Chemilumineszenz-Immunoassay zur Bestimmung eines Antikörpers in einer flüssigen Probe, umfassend die Schritte:
(a) Inkubieren der flüssigen Probe zusammen mit einem Antikörper, der spezifisch mit dem Antikörper in der flüssigen Probe reagiert und mit Biotin markiert ist, und einem Antigen, das spezifisch mit dem Antikörper in der flüssigen Probe reagiert und mit einer chemilumineszierenden Verbindung markiert ist, unter Bildung eines Immunkomplexes, der den markierten Antikörper, den Antikörper in der flüssigen Probe und das markierte Antigen umfaßt;
(b) Imprägnieren eines Glasfaseraggregats, an das Avidin oder Streptoavidin gebunden ist, mit einem Gemisch aus Schritt (a) und Inkubieren, um den Immunkomplex durch eine Avidin-Biotin-Reaktion an dem Glasfaseraggregat abzufangen;
(c) Abtrennen des Glasfaseraggregats, das immobilisierten Avidin-Immunkomplex trägt, aus dem Gemisch;
(d) Induzieren von Lumineszenz der chemilumineszierenden Verbindung in dem immobilisierten Avidin-Immunkomplex mit einem lumineszenzinduzierenden Mittel und Messen der Intensität der Lumineszenz des Glasfaseraggregats.

6. Chemilumineszenz-Immunoassay zur Bestimmung eines Antikörpers in einer flüssigen Probe, umfassend die Schritte:
(a) Inkubieren der flüssigen Probe zusammen mit einem ersten Antigen, das spezifisch mit dem Antikörper in der flüssigen Probe reagiert und mit Biotin markiert ist, und einem zweiten Antigen, das spezifisch mit dem Antikörper in der flüssigen Probe reagiert und mit einer chemilumineszierenden Verbindung markiert ist, unter Bildung eines Immunkomplexes, der das markierte erste Antigen, den Antikörper in der flüssigen Probe und das markierte zweite Antigen umfaßt;
(b) Imprägnieren eines Glasfaseraggregats, an das Avidin oder Streptoavidin gebunden ist, mit einem Gemisch aus Schritt (a) und Inkubieren, um den Immunkomplex durch eine Avidin-Biotin-Reaktion an dem Glasfaseraggregat abzufangen;
(c) Abtrennen des Glasfaseraggregats, das immobilisierten Avidin-Immunkomplex trägt, aus dem Gemisch;
(d) Induzieren von Lumineszenz der chemilumineszierenden Verbindung in dem immobilisierten Avidin-Immunkomplex mit einem lumineszenzinduzierenden Mittel und Messen der Intensität der Lumineszenz des Glasfaseraggregats.

7. Chemilumineszenz-Immunoassay gemäß einem der Ansprüche 1 bis 6, wobei Avidin oder Streptoavidin an das Glasfaseraggregat gebunden wird durch
(i) Bereitstellen eines Glasaggregats, das eine Aminogruppe trägt;
(ii) Umsetzen der Aminogruppe mit Biotin, an das eine N-Hydroxysuccinimidgruppe gebunden ist, so daß Biotin auf die Glasfaser aufgebracht wird; und
(iii) Umsetzen des aufgebrachten Biotins mit Avidin oder Streptoavidin.

8. Chemilumineszenz-Immunoassay gemäß einem der Ansprüche 1 bis 6, wobei Avidin oder Streptoavidin an das Glasfaseraggregat gebunden wird durch
(i) Bereitstellen eines Glasaggregats, das eine Aminogruppe trägt;
(ii) Umsetzen der Aminogruppe des Glasfaseraggregats mit Bernsteinsäureanhydrid, um eine Carbonsäuregruppe einzuführen; und
(iii) Umsetzen der eingeführten Carbonsäuregruppe mit Dicyclohexylcarbodiimid und N-Hydroxysuccinimid, so daß eine N-Hydroxysuccinimidgruppe auf die Glasfaser aufgebracht wird; und
(iv) Umsetzen der aufgebrachten N-Hydroxysuccinimidgruppe mit Avidin oder Streptoavidin.

## Revendications

1. Essai immunologique à chimioluminescence pour doser un antigène dans un échantillon fluide comprenant les étapes consistant à :
(a) incuber ledit échantillon fluide avec un premier anticorps qui réagit de manière spécifique avec ledit antigène et qui est marqué par la biotine, et avec un second anticorps qui réagit de manière spécifique avec ledit antigène et qui est marqué par un composé chimioluminescent, pour former un complexe immun comprenant ledit premier anticorps, ledit antigène et ledit second anticorps,
(b) imprégner un agrégat de fibre de verre sur lequel est liée de l'avidine ou de la streptoavidine, avec un mélange issu de l'étape (a), et l'incuber pour capturer ledit complexe immun sur ledit agrégat de fibre de verre via une réaction avidine-biotine,
(c) séparer dudit mélange ledit agrégat de fibre de verre portant le complexe immun lié à l'avidine immobilisée,
(d) induire de la luminescence à partir dudit composé chimioluminescent dans le complexe immun lié à l'avidine immobilisée, avec un agent inducteur de luminescence et mesurer l'intensité de la luminescence provenant dudit agrégat de fibre de verre.

2. Essai immunologique à chimioluminescence pour doser un antigène dans un échantillon fluide comprenant les étapes consistant à :
(a) incuber ledit échantillon fluide avec un anticorps qui réagit de manière spécifique avec ledit antigène et qui est marqué par un agent choisi parmi la biotine et un composé chimioluminescent, avec un antigène qui est marqué par l'autre agent parmi la biotine et le composé chimioluminescent, pour réaliser une réaction anticorps-antigène compétitive pour former un complexe immun comprenant ledit anticorps marqué et ledit antigène de l'échantillon fluide ou ledit antigène marqué,
(b) imprégner un agrégat de fibre de verre sur lequel est liée de l'avidine ou de la streptoavidine, avec un mélange issu de l'étape (a), et l'incuber pour capturer ledit complexe immun sur ledit agrégat de fibre de verre via une réaction avidine-biotine,
(c) séparer dudit mélange ledit agrégat de fibre de verre portant le complexe immun lié à l'avidine immobilisée,
(d) induire de la luminescence à partir dudit composé chimioluminescent dans le complexe immun lié à l'avidine immobilisée, avec un agent inducteur de luminescence et mesurer l'intensité de la luminescence provenant dudit agrégat de fibre de verre.

3. Essai immunologique à chimioluminescence pour doser un anticorps dans un échantillon fluide comprenant les étapes consistant à :
(a) incuber ledit échantillon fluide avec un antigène qui réagit de manière spécifique avec ledit anticorps et qui est marqué avec un agent choisi parmi la biotine et un composé chimioluminescent, et avec un anticorps qui réagit de manière spécifique avec ledit anticorps et qui est choisi parmi l'autre agent choisi parmi la biotine et le composé chimioluminescent, pour former un complexe immun comprenant ledit antigène, ledit anticorps de l'échantillon et ledit anticorps marqué,
(b) imprégner un agrégat de fibre de verre sur lequel est liée de l'avidine ou de la streptoavidine, avec un mélange issu de l'étape (a), et l'incuber pour capturer ledit complexe immun sur ledit agrégat de fibre de verre via une réaction avidine-biotine,
(c) séparer dudit mélange ledit agrégat de fibre de verre portant le complexe immun lié avidine immobilisée,
(d) induire de la luminescence à partir dudit composé chimioluminescent dans le complexe immun lié à l'avidine immobilisée, avec un agent inducteur de luminescence et mesurer l'intensité de la luminescence provenant audit agrégat de fibre de verre.

4. Essai immunologique à chimioluminescence pour doser un anticorps dans un échantillon fluide comprenant les étapes consistant à :
(a) incuber ledit échantillon fluide avec un antigène qui réagit de manière spécifique avec ledit anticorps et qui est marqué par un agent choisi parmi la biotine et un composé chimioluminescent, et avec un anticorps qui réagit de manière spécifique avec ledit antigène marqué et qui est marqué par l'autre agent parmi la biotine et le composé chimioluminescent, pour réaliser une réaction anticorps-antigène compétitive pour former un complexe immun comprenant ledit antigène et ledit anticorps de l'échantillon ou ledit anticorps marqué,
(b) imprégner un agrégat de fibre de verre sur lequel est liée de l'avidine ou de la streptoavidine, avec un mélange issu de l'étape (a), et l'incuber pour capturer ledit complexe immun sur ledit agrégat de fibre dc verre via une réaction avidine-biotine,
(c) séparer dudit mélange ledit agrégat de fibre de verre portant le complexe immun lié à l'avidine immobilisée,
(d) induire de la luminescence à partir dudit composé chimioluminescent dans le complexe immun lié à l'avidine immobilisé, avec un agent inducteur de luminescence et mesurer l'intensité de la luminescence provenant dudit agrégat de fibre de verre.

5. Essai immunologique à chimioluminescence pour doser un anticorps dans un échantillon fluide comprenant les étapes consistant à :
(a) incuber ledit échantillon fluide avec un anticorps qui réagit de manière spécifique avec ledit anticorps dans ledit échantillon fluide et qui est marqué par la biotine, et avec un antigène qui réagit de manière spécifique avec ledit anticorps dans ledit échantillon fluide et qui est marqué par un composé chimioluminescent, pour former un complexe immun comprenant ledit anticorps marqué, ledit anticorps de l'échantillon fluide et ledit antigène marqué,
(b) imprégner un agrégat de fibre de verre sur lequel est liée de l'avidine ou de la streptoavidine, avec un mélange issu de l'étape (a), et l'incuber pour capturer ledit complexe immun sur ledit agrégat de fibre de verre via une réaction avidine-biotine,
(c) séparer dudit mélange ledit agrégat de fibre de verre portant le complexe immun lié à l'avidine immobilisée,
(d) induire de la luminescence à partir dudit composé chimioluminescent dans le complexe immun lié à l'avidine immobilisée, avec un agent inducteur de luminescence et mesurer l'intensité de la luminescence provenant dudit agrégat de fibre de verre.

6. Essai immunologique à chimioluminescence pour doser un anticorps dans un échantillon fluide comprenant les étapes consistant à :
(a) incuber ledit échantillon fluide avec un premier antigène qui réagit de manière spécifique avec ledit anticorps dans ledit échantillon fluide et qui est marqué par la biotine et avec un second antigène qui réagit de manière spécifique avec ledit anticorps dans ledit échantillon fluide et qui est marqué par un composé chimioluminescent, pour former un complexe immun comprenant ledit premier antigène marqué, ledit anticorps de l'échantillon fluide et ledit second antigène marqué,
(b) imprégner un agrégat de fibre de verre sur lequel est liée de l'avidine ou de la streptoavidine, avec un mélange issu de l'étape (a), et l'incuber pour capturer ledit complexe immun sur ledit agrégat de fibre de verre via une réaction avidine-biotine,
(c) séparer dudit mélange ledit agrégat de fibre de verre portant le complexe immun lié à l'avidine immobilisée,
(d) induire de la luminescence à partir dudit composé chimioluminescent dans le complexe immun lié à l'avidine immobilisée, avec un agent inducteur de luminescence et mesurer l'intensité de la luminescence provenant dudit agrégat de fibre de verre.

7. Essai immunologique à chimioluminescence selon l'une quelconque des revendications 1 à 6, dans lequel l'avidine ou la streptoavidine est liée audit agrégat de fibre de verre en :
(i) préparant un agrégat de verre portant un groupe amino,
(ii) faisant réagir ledit groupe amino avec de la biotine à laquelle est lié un groupe N-hydroxysuccinimide, de façon à introduire la biotine sur ladite fibre de verre, et en
(iii) faisant réagir ladite biotine introduite avec de l'avidine ou de la streptoavidine.

8. Essai immunologique à chimioluminescence selon l'une quelconque des revendications 1 à 6, dans lequel l'avidine ou la streptoavidine est liée audit agrégat de libre de verre en :
(i) préparant un agrégat de verre portant un qroupe amino,
(ii) faisant réagir ledit groupe amino de l'agrégat de verre avec de l'anhydride succinique pour introduire un groupe carboxylique,
(iii) faisant réagir ledit groupe carboxyle introduit avec du dicyclohexylcarbodiimide et du N-hydroxysuccinimide de façon à introduire un groupe N-hydroxysuccinimide sur ladite fibre de verre, et en
(iv) faisant réagir ledit groupe N-hydroxy-succinimide introduit avec de l'avidine ou de la streptoavidine.
